# EUROPEAN PATENT SPECIFICATION

(11) **EP 2 133 692 B1**
(45) Date of publication and mention of the grant of the patent: **09.10.2013**
(21) Application number: 07806502.6
(22) Date of filing: 31.08.2007
(51) Int. Cl.: G01N 33/487, G01N 33/68, G01N 33/92, C12Q 1/26

(54) **DIAGNOSIS METHOD FOR FATTY LIVER DISEASE, DIAGNOSIS APPARATUS, DIAGNOSIS PROGRAM, DIAGNOSTIC AGENT, AND METHOD FOR SCREENING FOR THERAPEUTIC AGENT FOR FATTY LIVER DISEASE**
DIAGNOSEVERFAHREN FÜR FETTLEBERKRANKHEIT, DIAGNOSEVORRICHTUNG, DIAGNOSEPROGRAMM, DIAGNOSEMITTEL UND VERFAHREN ZUR SUCHE NACH EINEM THERAPEUTISCHEN MITTEL GEGEN FETTLEBERKRANKHEIT
PROCEDE, APPAREIL, PROGRAMME ET AGENT DE DIAGNOSTIC DE LA STEATOSE HEPATIQUE, ET PROCEDE DE CRIBLAGE D'AGENT THERAPEUTIQUE CONTRE LA STEATOSE HEPATIQUE

(30) Priority: 06.11.2006 JP 2006299978
(43) Date of publication of application: 16.12.2009
(73) Proprietor: Yokohama City University, Yokohama-shi Kanagawa 236-0027 (JP)
(72) Inventor: NAKAJIMA, Atsushi, Yokohama-shi Kanagawa 2360004 (JP); YONEDA, Masato, Yokohama-shi Kanagawa 2360004 (JP); FUJITA, Koji, Yokohama-shi Kanagawa 2360004 (JP); NAKAE, Dai, Yokohama-shi Kanagawa 2270055 (JP); TAKAMURA, Takeji, Atsugi-shi Kanagawa 2430005 (JP); TAKAHASHI, Masakazu, Ryugasaki-shi Ibaraki 3010043 (JP); HATANAKA, Yutaka, Tokorozawa-shi, Saitama 3590038 (JP)
(74) Representative: Woods, Geoffrey Corlett
(86) International application number: PCT/JP2007/067029
(87) International publication number: WO 2008/056480

(56) References cited:
- JP-A- 59 140 899
- HISE M K ET AL: "Determination of intracellular choline levels by an enzymatic assay" ANALYTICAL BIOCHEMISTRY, ACADEMIC PRESS INC, NEW YORK, vol. 135, no. 1, 1 November 1983 (1983-11-01), pages 78-82, XP024825851 ISSN: 0003-2697 [retrieved on 1983-11-01]
- NEHRA V. ET AL.: 'Nutritional and metabolic considerations in the etiology of nonalcoholic steatohepatitis' DIGESTIVE DISEASES AND SCIENCES vol. 46, no. 11, 2001, pages 2347 - 2352, XP003022529
- DUMAS M.-E. ET AL.: 'Metabolic profiling reveals a contribution of gut microbiota to fatty liver phenotype in insulin-resistant mice' PROC. NATL. ACAD. SCI. USA vol. 103, no. 33, 15 August 2006, pages 12511 - 12516, XP003022530

## Description

### Technical Field

The present invention relates to a method for diagnosing nonalcoholic fatty liver disease and a method for screening a therapeutic agent for nonalcoholic fatty liver disease.

### Background Art

Due to westernization of dietary habit in Japan and lack of exercise resulting from urbanization of life style in recent years, patients with fatty liver which progresses to steatohepatitis without drinking alcohol are rapidly increasing in number in the U.S. and European countries as well as in Japan. Such a new disease concept is called nonalcoholic fatty liver disease (NAFLD) or nonalcoholic steatohepatitis (NASH) (Ludwig, J., Viggiano, T.R., McGill, et al., Nonalcoholic steatohepatitis: Mayo Clinic experiences with a hitherto unnamed disease. Mayo Clin Proc 55, p.434-438, 1980). Dumas et al. PNAS (2006), Vol. 103, pp.12511-12516 discloses lowered choline levels on plasma from Mice with NAFLD. It is known that liver fibrosis gradually progresses to cirrhosis if NASH is left untreated. It is also known that liver cancer may develop from fatty liver in some cases. Fatty liver is an abnormality that is detected at a complete medical checkup or a health checkup with a high frequency. So far, it has been said that clinical conditions in patients with fatty liver having mildly abnormal liver functions who do not drink alcohol are of virtually no pathological significance and can be left untreated.

It has long been known that an alcohol drinking habit alone causes fatty liver or hepatopathy. Since the condition is caused by alcohol in this case, treatment involves cutting down or secession of alcohol drinking. On the other hand, not only causes of NASH or NAFLD with no alcohol drinking habit have not been identified, but also pathological conditions thereof have not been defined, and diagnostic methods and therapies have not been established.

Liver biopsy is currently the most reliable possible diagnostic method although it is a very invasive examination. Because of the high invasiveness of liver biopsy, development of a simpler examination method useful for diagnosis is awaited.

Furthermore, it is known that underlying obesity, diabetes, or the like often plays a role in pathogenesis of this disease. However, it is unknown how obesity, lack of exercise, and the like alone can progress the disease to cirrhosis. At least, it is thought that new diseases which hardly occurred before have emerged in recent years, and it is required to understand pathological conditions thereof.

For review of fatty liver disease, John B. Dixon et al., Gastroenterology 2001, p.121, 91-100 and Gastroenterology 2002, 123, p.1705-1725 can be referred to.

Nehra et al, (Digestive Diseases and Sciences (2001) 46: 2347-2352) reports that increased serum concentrations of three fatty acids were seen in non-alcoholic steatohepatitis (NASH) patients and were associated with the development of more severe liver disease. Hise et al, (Analytical Biochemistry (1983) 135: 78-82) describes an enzymatic assay for the measurement of intracellular choline. JP-A-59 140899 describes a method for determination of a substrate by reacting the substrate with an oxidase and detecting the produced superoxidase ion.

### Disclosure of the Invention

### Problems to be Solved by the Invention

Accordingly, an object of the present invention is to provide a method for diagnosing fatty liver disease which is simpler than conventional liver biopsy and can determine the presence of NAFLD, the degree of progression of a condition (may also be referred to as severity or progression process in the following description), or the like. Another object of the present invention is to provide a method for a screening therapeutic agent for NAFLD.

### Means for Solving the Problems

Based on a rat model given a choline-deficient diet which develops fatty liver or steatohepatitis progressing to cirrhosis or liver cancer, the present inventors formed a hypothesis that choline deficiency may also be present in humans who have NASH or NAFLD and used a gas chromatography mass spectrometer to quantify the contents of choline, betaine, phosphatidylcholine, and sphingo myelin in serum in blood-derived samples of patients definitely diagnosed as NASH or NAFLD by liver biopsy. Unexpectedly, the results showed that choline levels in the blood-derived samples significantly differ between healthy subjects and patients with fatty liver disease as well as among fatty liver disease patients with different degrees of progression. This result strongly suggests that fatty liver disease can be simply diagnosed, and the effect of a treatment can be evaluated by measuring the concentration of choline contained a blood-derived sample. The present invention was accomplished based on this finding.

The present invention is summarized as follows.
(1) A method for diagnosing non alcoholic fatty liver disease, comprising the steps of (a) measuring the concentration of choline contained in a blood-derived sample from a subject; and (b) determining the presence of non alcoholic fatty liver disease based on the choline concentration wherein the subject is classified as a patient with non alcoholic fatty liver disease based on the presence of an increased choline concentration during the determination step when compared to the choline concentration in a blood-derived sample from a healthy subject or from a patient with alcoholic steatohepatitis.
(2) The method according to (1), characterized in that the blood-derived sample is a serum sample, and the choline concentration is the concentration of free choline in serum.
(3) The method according to (1), characterized in that the choline concentration is measured by high performance liquid chromatography, liquid chromatography-mass spectrometry, use of enzyme reagents including choline esterase, use of a reagent which chemically reacts with choline, electrophoresis, nuclear magnetic resonance, ultracentrifugation, spectroscopy using an ultraviolet ray, or measurement of a potential difference.
(4) The method according to any one of (1) to (3) wherein a subject is classified as a patient with non alcoholic fatty liver disease based on the presence of a choline concentration significantly exceeding the mean + confidence interval of the choline concentration in a blood-derived sample from a normal individual.
(5) The method according to (1), characterized in that the degree of progression of non alcoholic fatty liver disease in the subject is determined based on the choline concentration during the determination step.
   In the method for diagnosing non alcoholic fatty liver disease according to (5), the degree of progression of non alcoholic fatty liver disease may be defined as type 1, in which non alcoholic simple fatty liver is present, type 2, in which non alcoholic steatohepatitis is present, type 3, in which non alcoholic fatty liver necrosis is present, or type 4, in which hepatocyte necrosis associated with Mallory body formation or fibrosis is present.
(6) The method according to (1), characterized in that the subject is classified as a patient with non alcoholic steatohepatitis and is further classified for the degree of progression of nonalcoholic steatohepatitis based on the choline concentration.
   In the method according to (6), the degree of progression may be defined by stages 1 to 4 according to a classification of fibrosis in nonalcoholic steatohepatitis.
   In the method according to (6), the degree of progression may be defined by necrosis/inflammation grades 1 to 3 in nonalcoholic steatohepatitis.
(7) The method according to (1), characterized in that the subject is classified as a patient with nonalcoholic fatty liver disease, nonalcoholic simple fatty liver, or nonalcoholic steatohepatitis based on the choline concentration during the determination step.
(8) The method according to (1), characterized in that the subject is suspected of having steatohepatitis and is classified as a patient with nonalcoholic steatohepatitis based on the presence of an increased choline concentration during the determination step when compared to the choline concentration in a blood-derived sample from a healthy subject or from a patient with alcoholic steatohepatitis.
(9) A method for screening a therapeutic agent for fatty nonalcoholic liver disease, comprising the steps of comparing the concentration of choline contained in a blood sample from a subject before and after a test substance has been administered; and identifying a test substance which significantly decreases the choline concentration as a therapeutic agent for nonalcoholic fatty liver disease.

In the method for screening a therapeutic agent for non alcoholic fatty liver disease according to (9), the blood-derived sample may be a serum sample, and the choline concentration may be the concentration of free choline in serum.

In the method for screening a therapeutic agent for non alcoholic fatty liver disease according to (9), the choline concentration may be measured by high performance liquid chromatography, liquid chromatography-mass spectrometry, use of enzyme reagents including choline esterase, use of a reagent which chemically reacts with choline, electrophoresis, nuclear magnetic resonance, ultracentrifugation, spectroscopy using an ultraviolet ray, or measurement of a potential difference.

Also described herein are:
(10) An apparatus for diagnosing fatty liver disease, comprising measuring means for measuring the concentration of choline contained in a blood-derived sample collected from a subject; input means for inputting the choline concentration measured by the measuring means; and computing means for determining the presence of fatty liver disease, severity of the disease, or a therapeutic effect on the disease based on the choline concentration inputted from the input means.
(11) The apparatus for diagnosing fatty liver disease according to (10), characterized in that the blood-derived sample is a serum sample, and the concentration of free choline in serum is measured by the measuring means.
(12) The apparatus for diagnosing fatty liver disease according to (10), characterized in that the measuring means is a high performance liquid chromatography apparatus, a liquid chromatography-mass spectrometer, an enzyme reagent reaction apparatus including choline esterase, a chemical reagent reaction apparatus including a reagent which chemically reacts with choline, an electrophoresis apparatus, a nuclear magnetic resonance apparatus, an ultracentrifugation apparatus, a spectrometer using an ultraviolet ray, or potential difference measuring apparatus.
(13) The apparatus for diagnosing fatty liver disease according to (10), characterized in that the computing means classifies the subject as a healthy subject or a patient with fatty liver disease based on the choline concentration.
(14) The apparatus for diagnosing fatty liver disease according to (10), characterized in that the computing means determines the degree of progression of fatty liver disease in the subject based on the choline concentration.
(15) The apparatus for diagnosing fatty liver disease according to (14), characterized in that the degree of progression of fatty liver disease is defined as type 1, in which nonalcoholic simple fatty liver is present, type 2, in which nonalcoholic steatohepatitis is present, type 3, in which nonalcoholic fatty liver necrosis is present, or type 4, in which hepatocyte necrosis associated with Mallory body formation or fibrosis is present.
(16) The apparatus for diagnosing fatty liver disease according to (10), characterized in that the computing means further classifies the subject classified as a patient with nonalcoholic steatohepatitis for the degree of progression of nonalcoholic steatohepatitis based on the choline concentration.
(17) The apparatus for diagnosing fatty liver disease according to (16), characterized in that the degree of progression is defined by stages 1 to 4 according to a classification of fibrosis in nonalcoholic steatohepatitis.
(18) The apparatus for diagnosing fatty liver disease according to (16), characterized in that the degree of progression is defined by necrosis/inflammation grades 1 to 3 in nonalcoholic steatohepatitis.
(19) The apparatus for diagnosing fatty liver disease according to (10), characterized in that the computing means classifies the subject as a healthy subject or a patient with nonalcoholic fatty liver disease, nonalcoholic simple fatty liver, or nonalcoholic steatohepatitis based on the choline concentration.
(20) The apparatus for diagnosing fatty liver disease according to (10), characterized in that the computing means classifies the subject suspected of having steatohepatitis as a patient with alcoholic steatohepatitis or nonalcoholic steatohepatitis based on the choline concentration.
(21) A program for diagnosing fatty liver disease, executing a step in which input means inputs the concentration of choline contained in a blood-derived sample collected from a subject into a computer having the input means and computing means; and a step in which the computing means determines the presence of fatty liver disease, severity of the disease, or a therapeutic effect on the disease based on the choline concentration inputted in the step above.
(22) The program for diagnosing fatty liver disease according to (21), characterized in that the blood-derived sample is a serum sample, and the input means inputs the concentration of free choline in serum.
(23) The program for diagnosing fatty liver disease according to (21), characterized in that the input means inputs a choline concentration outputted from a high performance liquid chromatography apparatus, a liquid chromatography-mass spectrometer, an enzyme reagent reaction apparatus including choline esterase, a chemical reagent reaction apparatus including a reagent which chemically reacts with choline, an electrophoresis apparatus, a nuclear magnetic resonance apparatus, an ultracentrifugation apparatus, a spectrometer using an ultraviolet ray, or a potential difference measuring apparatus, or a choline concentration outputted from a recording apparatus which records a choline concentration measured by the apparatus.
(24) The program for diagnosing fatty liver disease according to (21), characterized in that the computing means classifies a subject as a healthy subject or a patient with fatty liver disease based on the choline concentration.
(25) The program for diagnosing fatty liver disease according to (21), characterized in that the computing means determines the degree of progression of fatty liver disease in the subject based on the choline concentration.
(26) The program for diagnosing fatty liver disease according to (25), characterized in that the degree of progression of fatty liver disease is defined as type 1, in which nonalcoholic simple fatty liver is present, type 2, in which nonalcoholic steatohepatitis is present, type 3, in which nonalcoholic fatty liver necrosis is present, or type 4, in which hepatocyte necrosis associated with Mallory body formation or fibrosis is present.
(27) The program for diagnosing fatty liver disease according to (22), characterized in that the computing means further classifies the subject classified as a patient with nonalcoholic steatohepatitis for the degree of progression of nonalcoholic steatohepatitis based on the choline concentration.
(28) The program for diagnosing fatty liver disease according to (27), characterized in that the degree of progression is defined by stages 1 to 4 according to a classification of fibrosis in nonalcoholic steatohepatitis.
(29) The program for diagnosing fatty liver disease according to (27), characterized in that the degree of progression is defined by necrosis/inflammation grades 1 to 3 in nonalcoholic steatohepatitis.
(30) The program for diagnosing fatty liver disease according to (21), characterized in that the computing means classifies the subject as a healthy subjects or a patient with nonalcoholic fatty liver disease, nonalcoholic simple fatty liver, or nonalcoholic steatohepatitis based on the choline concentration.
(31) The program for diagnosing fatty liver disease according to (21), characterized in that the computing means classifies the subject suspected of having steatohepatitis as a patient with alcoholic steatohepatitis or nonalcoholic steatohepatitis based on the choline concentration.
(32) An agent for diagnosing fatty liver disease, comprising choline oxidase, which produces betaine and hydrogen peroxide using a choline component as a substrate; and a detection reagent which detects hydrogen peroxide produced by the choline oxidase.
(33) The agent for diagnosing fatty liver disease according to (32), characterized in that the detection reagent comprises peroxidase, a coupler, and a hydrogen donor.
(34) The agent for diagnosing fatty liver disease according to (32), characterized by determining the degree of progression of fatty liver disease.
(35) The agent for diagnosing fatty liver disease according to (34), characterized in that the degree of progression of fatty liver disease is defined as type 1, in which nonalcoholic simple fatty liver is present, type 2, in which nonalcoholic steatohepatitis is present, type 3, in which nonalcoholic fatty liver necrosis is present, or type 4, in which hepatocyte necrosis associated with Mallory body formation or fibrosis is present.
(36) The agent for diagnosing fatty liver disease according to (32), characterized by further classifying a subject classified as having nonalcoholic steatohepatitis for the degree of progression of nonalcoholic steatohepatitis.
(37) The agent for diagnosing fatty liver disease according to (36), characterized in that the degree of progression is defined by stages 1 to 4 according to a classification of fibrosis in nonalcoholic steatohepatitis.
(38) The agent for diagnosing fatty liver disease according to (36), characterized in that the degree of progression is defined by necrosis/inflammation grades 1 to 3 in nonalcoholic steatohepatitis.
(39) The agent for diagnosing fatty liver disease according to (32), characterized by classifying the subject as a healthy subject or a patient with nonalcoholic fatty liver disease, nonalcoholic simple fatty liver, or nonalcoholic steatohepatitis.
(40) The agent for diagnosing fatty liver disease according to (32), characterized by classifying the subject suspected of having steatohepatitis as a patient with alcoholic steatohepatitis or nonalcoholic steatohepatitis.

### Advantage of the Invention

The present invention has enabled the diagnosis of NAFLD, steatohepatitis and the determination of a therapeutic effect on such a disease by a method that is simpler, safer, and more inexpensive than conventional liver biopsy.

### Brief Description of the Drawings

Figure 1 is a configuration diagram schematically showing the configuration of an analyzing apparatus to which the present invention is applied;
Figure 2 is a characteristic diagram showing serum choline concentrations in patients with diseases;
Figure 3 is a characteristic diagram showing schemata of choline resistance;
Figure 4 is a characteristic diagram showing serum choline concentrations in healthy subjects, patients with simple fatty liver, and patients with NASH;
Figure 5 is a characteristic diagram showing serum choline concentrations in patients with NASH of different severity;
Figure 6 is a characteristic diagram showing the results of measurement of serum choline concentrations using enzyme reagents in normal subjects, patients with simple fatty liver, patients with NASH at F1, F2, and F3, and patients with ASH;
Figure 7 is a characteristic diagram showing the results of evaluation of the enzyme reagents used in the Examples for the ability of measuring choline concentrations; and
Figure 8 is a characteristic diagram showing correlations between the results of measurement of choline concentrations using an analyzing apparatus and the results of measurement of choline concentrations using enzyme reagents.

### Best Mode for Carrying Out the Invention

Hereafter, the present invention will be explained in detail.

The diagnostic method of the present invention comprises steps of measuring the concentration of choline contained in a blood-derived sample collected from a subject and determining the presence of nonalcoholic fatty liver disease, severity of the disease, or a therapeutic effect on the disease based the choline concentration as defined on the claims. In particular, this diagnostic method is not directly or indirectly invasive, but involves treatment of a blood-derived sample collected from the subject. The term "blood-derived sample" used herein encompasses a blood sample collected from a subject and a serum sample separated from the blood sample and prepared. In the present invention, a serum sample is preferably used to improve precision of analysis of the choline concentration described later. For example, a serum sample can be separated by placing a blood sample collected from a subject in a glass tube containing a separating agent, and subjecting it to centrifugation, for example, at 2000 rpm for 10 minutes.

Furthermore, a blood sample is preferably collected from a subject in fasting as in other laboratory tests or the like. Furthermore, to collect blood from a subject, it is recommended to collect 5 mL of venous blood from the forearm or the elbow. The blood collecting method and the volume of blood collected may be suitably changed. Furthermore, prohibition of eating and drinking after an appropriate time on the day before blood collection (for example, 8.00 p.m.) improves reproducibility and reliability of data.

To measure the choline concentration in a blood-derived sample, conventional known techniques can be used. The structural formula of choline is as follows:

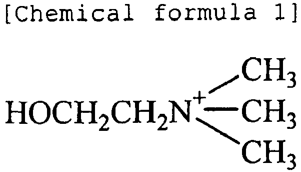

### Method for measuring choline concentrations 1

The choline concentration in a blood-derived sample can be measured by using an HPLC-mass spectrometer according to, for example, the method of Koc, H., et al., (Anal. Che. 2002, 74, 4734-4740). It is recommended to measure choline by the isotope dilution method. In the measurement by the isotope dilution method, it is recommended to dilute a choline preparation labeled with a stable isotope to create a calibration curve. It is recommended to use a phosphate buffer or the like for dilution.

### Method for measuring choline concentrations 2

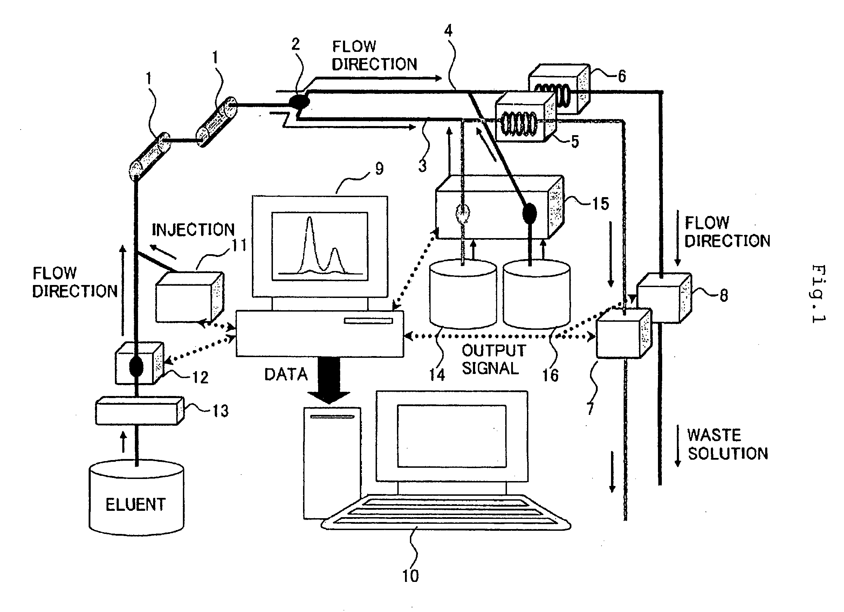

Furthermore, to measure the choline concentration in a blood-derived sample, the analyzing method, the analyzing apparatus, and the analyzing program disclosed in Japanese Patent No. 3899041 can be used. According to these analyzing method, analyzing apparatus, and analyzing program, lipoprotein components contained in a sample are separated depending on the particle size, and cholesterol components and triglyceride components contained in the separated lipoprotein components are quantified. In the application thereof to the present invention, it is sufficient to use reagents for measuring phospholipids and free choline instead of the reagents for measuring cholesterols and triglycerides, so that free choline observed in a region with a slow elution time should be measured. Specifically, for example, as shown in Figure 1, the analyzing apparatus disclosed in Japanese Patent No. 3899041 is equipped with a column 1 which can separate lipoprotein components contained in a test sample, a splitter 2 which divides an eluent containing lipoproteins eluted from the column 1 into two flows, a first passage 3 and a second passage 4 divided by the splitter 2, a cholesterol (hereinafter, referred to as "TC") reaction unit 5 positioned in the first passage 3, a triglyceride (hereinafter, referred to as "TG") reaction unit 6 positioned in the second passage 4, a TC detection unit 7 positioned downstream of the TC reaction unit 5 in the first passage 3, a TG detection unit 8 positioned downstream of the TG reaction unit 6 in the second passage 4, a system controller 9 which controls operations of this apparatus and into which signals are inputted from the TC detection unit 7 and the TG reaction unit 8, and a computing apparatus 10 connected to the system controller 9. In the application thereof to the diagnostic method of the present invention, a phospholipid measuring agent which can measure the choline concentration in a sample is used instead of the TC reaction unit 5 and the TC detection unit 7 or the TG reaction unit 6 and the TG detection unit 8. As the phospholipid measuring agent, Liquid PL (trade name) manufactured by Toyobo Co., Ltd. can be used.

It is noted that this lipoprotein analyzing apparatus is equipped with a sampler 11 which supplies a serum sample to the column 1, a first pump 12 for supplying an eluent to the column 1, and a degasser 13 which removes gasses from the eluent supplied by the first pump 12 to column 1. Furthermore, in this lipoprotein analyzing apparatus, the TC reaction unit 5 is linked via a second pump 15 to a reagent tank for TC 14 containing a reagent for quantifying TC contained in an eluent containing lipoproteins eluted from the column 1. TG reaction unit 6 is linked via the second pump 15 to a reagent tank for TG 16 containing a reagent for quantifying TG contained in an eluent containing lipoproteins eluted from the column 1. The TC reaction unit 5 and the TG reaction unit 6 are equipped with a reaction coil for controlling the temperature of a reaction of the above-described reagents and TC or TG, respectively.

The TC detection unit 7 is equipped with, for example, an ultraviolet visible light detector for detecting the absorbance of a reaction product produced by reaction of TC and the reagent in the TC reaction unit 5. Furthermore, the TG detection unit 8 is equipped with, for example, an ultraviolet visible light detector for detecting the absorbance of a reaction product produced by reaction of TG and the reagent in the TG reaction unit 6. Therefore, an ultraviolet visible light detector can also be used for measurement of the choline concentration in a sample using this apparatus. When the above-described reagents are used, it is sufficient to set the measurement wavelength of the ultraviolet visible detector as 590 to 610 nm, for example.

The system controller 9 has functions of receiving an output signal from the TC detection unit 7 or the TG detection unit 8 and outputting a chromatogram of TC or TG based on this signal as a result. Therefore, in the measurement of the choline concentration in a sample using this apparatus, a chromatogram of choline can be displayed with the elution time (min) on the horizontal axis and the detected values (mV) on the vertical axis.

### Method for measuring choline concentrations 3

Meanwhile, a method using enzyme reagents which can quantify the choline component contained in a blood-derived sample can also be applied to the measurement of the choline concentration in the blood-derived sample. The measurement principle involves conversion of choline to betaine by a choline oxidase reaction and quantification of hydrogen peroxide produced at the same time. Specifically, the reagents used in this method for measuring the choline concentration in a blood-derived sample comprise a choline oxidase which produces betaine and hydrogen peroxide using the choline component as a substrate and a detection reagent which detects hydrogen peroxide produced by the choline oxidase. The choline oxidase used in this method is not particularly limited with regard to the origin thereof, and examples thereof include choline oxidases derived from microorganisms such as, for example; *Arthrobacter globiformis* and the genus *Alcaligenes*. Furthermore, enzymes obtained by isolating genes thereof by gene manipulation, introducing them into another microorganism, and expressing them or modified enzymes obtained by chemically modifying them, or enzymes obtained by modifying these genes and expressing them or modified enzymes obtained by chemically modifying them can be used. The concentration of choline oxidase is, for example, 0.1 to 100 U/ml, preferably 1.0 to 40 U/mL.

The produced hydrogen peroxide is measured using a known hydrogen peroxide detection system. Representative examples of the system include a system in which a product of oxidative condensation of a coupler and a hydrogen donor is produced in the presence of peroxidase to induce coloration, and absorbance thereof is measured. Examples of the above-mentioned coupler include 4-aminoantipyrine or 3-methyl-2-benzobenzothiazolinone-hydrazone hydrochloride. Examples of the hydrogen donor include derivatives of aniline, anisidine, and toluidine (for example, N-ethyl-N-(2-hydroxy-3-sulfopropyl)-m-toluidine [TOOS], N-ethyl-N-(3-sulfopropyl)-m-anisidine [ADPS], N-ethyl-N-(3-sulfopropyl)aniline [ALPS], N-ethyl-N-(3-sulfopropyl)-3-methylaniline [TOPS], N-ethyl-N-(2-hydroxy-3-sulfopropyl)-3-methoxyaniline [ADOS], N-ethyl-N-(2-hydroxy-3-sulfopropyl)-3,5-dimethoxyaniline [DAOS], N-(2-hydroxy-3-sulfopropyl)-3,5-dimethoxyaniline [HDAOS], and N-ethyl-N-(2-hydroxy-3-sulfopropyl)-3,5-dimethylaniline [MAOS]).

Buffers used for the above-mentioned reaction are not particularly limited so long as they can maintain a buffering ability in a required pH range, and examples thereof include tris buffers, phosphate buffers, borate buffers, carbonate buffers, and GOOD buffers. Examples of the GOOD buffers include N-(2-acetamide)-2-aminoethanesulfonic acid (ACES), N,N-bis(2-hydroxyethyl)-2-aminoethanesulfonic acid (BES), N-cyclohexyl-2-aminoethanesulfonic acid (CHES), 2-[4-(2-hydroxyethyl)-1-piperazinyl]ethanesulfonic acid (HEPES), 2-morpholinoethanesulfonic acid (MES), piperazine-1,4-bis(2-ethanesulfonic acid) (PIPES), N-tris(hydroxymethyl)methyl-2-aminomethanesulfonic acid (TES), N-cyclohexyl-3-aminopropanesulfonic acid (CAPS), N-cyclohexyl-2-hydroxy-3-aminopropanesulfonic acid (CAPSO), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-hydroxy-3-[4-(2-hydroxyethyl)-1-piperazinyl]propanesulfonic acid (HEPPSO), 3-morpholinopropanesulfonic acid (MOPS), 2-hydroxy-3-morpholinopropanesulfonic acid (MOPSO), piperazine-1,4-bis(2-hydroxy-3-propanesulfonic acid) (POPSO), N-tris(hydroxymethyl)methyl-3-aminopropanesulfonic acid (TAPSO), N-(2-acetamide)iminodiacetic acid (ADA), N,N-bis(2-hydroxyethyl)glycine (bicine), N-[tris(hydroxymethyl)methyl]glycine (tricine). The pH of the buffer is adjusted within a range of 5 to 10, preferably 6.5 to 8.

Furthermore, to avoid influence of coexisting substances in a sample or the like, the reagents used in this method are preferably provided separately as a first reagent containing a detection reagent which detects hydrogen peroxide and a second reagent containing choline oxidase. In measurement of the choline concentration in a sample using enzyme reagents prepared as the first reagent and the second reagent, first, a first reaction is performed using the first reagent, and absorbance is measured after completion of the reaction. The first reaction can induce background coloration by the hydrogen peroxide component or the like contained in the sample. Subsequently, the second reagent is added to perform a second reaction, and absorbance is measured after completion of the reaction. Light emitted by hydrogen peroxide produced from choline in the sample alone can be obtained by subtracting the absorbance measured after completion of the first reaction from the absorbance measured after completion of the second reaction.

Various known avoiding agents are preferably added to the first reagent and the second reagent. For example, to avoid influence of ascorbic acid in a blood-derived sample, an ascorbic acid oxidase is preferably added to the first reagent. When an ascorbic acid oxidase is added to the first reagent, ascorbic acid can be eliminated from the blood-derived sample.

Furthermore, a stabilizer and the like are preferably added to these reagents to maintain stability for a long period. It is sufficient to select a stabilizer from common known stabilizers that have been shown to stabilize not only the enzymes used in the present invention but also other enzymes, and examples thereof include various proteins such as BSA, various saccharides, various sugar alcohols, various antimicrobial agents, various salts, various buffers, and various surfactants. These stabilizers can also be used in combination.

Furthermore, the reagents may be made so that several kinds of reagents such as, for example, lyophilized components contained in vials may be dissolved using a dissolving solution, and are prepared as the first reagent and the second reagent just before use, or can be used as they are as liquid reagents that do not require preparation.

Furthermore, the above-described first reagent and second reagent have favorable stability and can maintain performance thereof under refrigeration for at least 12 months as liquid reagents that do not require preparation.

The above-described measuring methods 1 to 3 are based on slightly different measurement principles, but measurement results thereof correlate to each other. Therefore, when choline concentrations are measured in the present invention, any of these measuring methods may be used. Furthermore, the concentration of choline contained in a blood-derived sample can be measured not only by using the above-described measuring methods, but also by suitably using, for example, techniques using a reagent which chemically reacts with choline, techniques using a gas chromatography-mass spectrometer, techniques using electrophoresis (refer to Biosci. Biotechnol. Biochem. Vol.65, No.11, p.2573-2576 [2001] and J. Chromatogr. Vol.343, No.1, p.186-189 [1985]), methods using NMR (refer to Anal. Biochem. Vol.137, No.2, p.324-329 [1984] and Radiat. Med. Vol.22, No.3, p.148-154 [2004]), methods using an ultracentrifuge, spectroscopy using an ultraviolet ray (refer to Electrophoresis Vol.17, No.10, p.1622-1626 [1996]), methods comprising measuring a potential difference (refer to Analyst. Vol.125, No.7, p.1281-1284 [2000]), and the like.

After the concentration of choline contained in a blood-derived sample collected from a subject is measured as described above, the possibility of the presence of fatty liver disease in a subject can be determined based on the measured choline concentration.

Here, fatty liver disease is a disease defined based on the past clinical diagnoses and a collective term for diseases which lead to hepatopathy due to deposition of triglycerides in hepatocytes (refer to The Japan Society of Hepatology, ed. "Clinical Practice Guidelines for NASH/NAFLD," 3rd ed. Bunkodo, October 7, 2006). Furthermore, fatty liver disease is classified into alcoholic fatty liver disease (AFLD) in patients having an alcohol drinking history and nonalcoholic fatty liver disease (NAFLD) in patients without any alcohol drinking history. Furthermore, fatty liver disease is roughly classified into simple fatty liver associated with only fat deposition in hepatocytes observed in tissue diagnosis and steatohepatitis associated with necrosis, inflammation, or fibrosis in addition to fatty change. Therefore, a patient who has no clear alcohol drinking history and shows steatohepatitis associated with necrosis, inflammation, or fibrosis in liver tissues is classified as a patient with nonalcoholic steatohepatitis (NASH). A patient who has a clear alcohol drinking history and shows steatohepatitis associated with necrosis, inflammation, or fibrosis in liver tissues is classified as a patient with alcoholic steatohepatitis (ASH). That is, NASH is severe NAFLD, and ASH is severe AFLD.

Furthermore, NAFLD is classified into four types by Matteori et al. (Matteori CA et al., Gastroenterology 116, p.1413-1419 [1999]). According to this classification, NAFLD is classified into type 1, in which simple fatty liver is present, type 2, in which steatohepatitis is present, type 3, in which fatty liver necrosis is present (associated with ballooning degeneration), and type 4, in which hepatocyte necrosis associated with Mallory body formation or fibrosis is present. In particular, types 3 and 4 in the classification of Matteori et al. may be defined as NASH.

Furthermore, the degree of progression of NASH is classified into four stages by Brunt et al. depending on the degree of fibrosis (Brunt EM et al., Amr J Gastroenterol 94:2467-2474(1999)). According to this classification, NASH is classified into stage 1, in which fibrosis is present in the centrilobular region (Zone 3) of the liver, stage 2, in which fibrosis is present in the portal vein region in addition to the centrilobular region (Zone 3) of the liver, stage 3, in which fibrosis associated with crosslink formation is further observed in the portal vein region, and stage 4, in which cirrhosis is present.

Furthermore, the degree of progression of NASH is defined by grades 1 to 3 by Brunt et al. depending on the degree of necrosis or inflammation (Brunt EM et al., Amr J Gastroenterol 94:2467-2474(1999)). The grade 1 in this classification refers to mild necrosis or inflammation, a condition represented by 66% or less fatty liver (primarily macrovesicular), in which mild hepatocyte ballooning around the central veins are observed, infiltration of mild inflammatory cells is not observed in the lobule, and mild inflammation is observed in the portal vein region. The grade 2 in this classification refers to moderate necrosis or inflammation, a condition in which fatty liver is present regardless of the degree, mild hepatocyte ballooning around the central veins is marked, lobular neutrophil cell infiltration associated with fibrosis around the central veins is observed, and mild to moderate infiltration of inflammatory cells is observed in the portal vein region. The grade 3 in this classification refers to severe necrosis or inflammation, a condition in which fatty liver is spread over the whole lobule, hepatocyte ballooning and clear disarray (around the central veins) are observed, hepatocyte ballooning associated with infiltration of neutrophil cells and mild chronic inflammation are observed, mild to moderate infiltration of inflammatory cells is observed in the portal vein region.

According to the diagnostic method of the present invention, a subject can be classified as a patient with nonalcoholic fatty liver disease, or by the degree of progression of nonalcoholic fatty liver disease (the above-described classifications by Matteori et al. and Brunt et al.). Specifically, when the concentration of choline contained in a blood-derived sample collected from a subject significantly exceeds a reference value, the subject can be diagnosed as nonalcoholic fatty liver disease or suspected nonalcoholic fatty liver disease. Here, the mean value and the confidence interval of choline concentrations in patients definitely diagnosed as fatty liver disease are calculated, and the "reference value" can be set as the lower limit value of the confidence interval. In the diagnostic method of the present invention, the reference value is not particularly limited, and a value significantly exceeding the mean and the confidence interval of the choline concentration in the healthy subjects can also be selected. Furthermore, the reference value varies depending on the above-described method for measuring choline concentrations, and cannot be defined uniquely. Furthermore, the reference value may vary depending on race, sex, age, past illnesses, and the like, and several values corresponding to race, sex, age, past illnesses, and the like may be may be prepared. For example, Alan L. Buchman et al., Journal of the American College of Nutrition, Vol. 18, No. 6, 598-601 (1999) can be referred to for serum choline concentrations in European and American patients.

Furthermore, according to the diagnostic method of the present invention, a subject can be classified as a patient with simple fatty liver or steatohepatitis. In this case as well, a reference value between healthy subjects and patients with simple fatty liver and a reference value between patients with simple fatty liver and patients with steatohepatitis are set. This diagnosis is based on the novel finding in the present invention that concentrations of choline contained in blood-derived samples increase in healthy subjects, patients with simple fatty liver, and patients fatty liver disease in this order.

Furthermore, according to the diagnostic method of the present disclosure, a subject suspected of steatohepatitis can also be classified as a patient with NASH based on the concentration of choline contained in a blood-derived sample collected from the subject. Here, the subject suspected of steatohepatitis is an individual suspected of having fatty liver disease by a technique used in conventional laboratory tests (for example, abdominal echo imaging or liver biopsy). When fatty liver disease is suspected in a conventional laboratory test, an alcohol drinking history is examined during history taking from the subject. When the subject has an alcohol drinking history, ASH is diagnosed, and when the subject has no alcohol drinking history, NASH is diagnosed. In the diagnostic method of the present disclosure, the subject can be classified as a patient with NASH not according to ambiguous criteria such as history taking, but based on objective numerical values such as concentrations of choline contained in blood-derived samples. This diagnosis is based on the novel finding in the present disclosure that concentrations of choline contained in blood-derived samples in patients with ASH are as low as those in healthy subjects, and those in patients with NASH are high.

Furthermore, according to the diagnostic method of the present disclosure, the progression process in patients with NASH (the same meaning as, for example, four grades of Brunt et al., or severity and the degree of progression of disease) can be determined. This diagnosis is based on the novel finding in the present disclosure that concentrations of choline contained in blood-derived samples correlate to the progression process, in particular, the degree of progression of liver fibrosis in patients with NASH. In this diagnosis, a patient with NASH may be classified as any of stages 1 to 4 corresponding to the above-described four-stage classification of Brunt et al. based on the concentration of choline contained in a blood-derived sample, but classification is not limited to this classification. In this diagnosis, NASH may be classified as type 3 or 4 in the above-described classification of Matteori et al. In this diagnosis, NASH may be classified as any of grades 1 to 3 corresponding to the above-described degrees of necrosis or inflammation of Brunt et al. Alternatively, the degree of progression of fibrosis in NASH is newly classified into several stages depending on the choline concentration beforehand based on the above-described novel findings of the present disclosure, and then patients with NASH can be diagnosed according to this new classification.

As described above, according to the diagnostic method of the present invention, diagnosis of nonalcoholic fatty liver disease and determination of severity thereof can be easily achieved based on the concentration of choline contained in a blood-derived sample. In particular, the diagnostic method of the present invention ensures a simple and highly objective examination which does not suffer from difficulty in procedures such as biopsy and abdominal echo imaging or dose not use ambiguous criteria such as history taking. In particular, an advantage of the method using enzyme reagents (method for measuring choline concentrations 3) among the above-described methods for measuring choline concentrations is that blood choline concentrations useful for diagnosis of NASH can be measured in the measurement range from approx. 0.1 mg/dL to 100 mg/dL or higher.

Furthermore, this diagnostic method may be used in combination with other diagnosis criteria (for example, absence of alcohol drinking history, diagnosis of fatty liver by diagnostic imaging methods [echo imaging, CT, MRI, etc.], exclusion of other diseases [for example, viral hepatitis, autoimmune hepatitis, etc.]) to establish a definite diagnosis.

Furthermore, according to the novel findings on which this diagnostic method is based, therapeutic agents for nonalcoholic fatty liver disease can be screened. Specifically, concentrations of choline contained in blood-derived samples before and after action of test substances to be screened are compared. When the concentrations of choline contained in blood-derived samples after action significantly decrease, the test substance is identified as a candidate substance for a therapeutic agent for nonalcoholic fatty liver disease. Furthermore, it is inferred that the higher the choline concentration decreasing rate is, the higher a therapeutic effect on fatty liver disease is. According to this screening method, a therapeutic agent for at least one type of fatty liver disease selected from the group consisting of nonalcoholic fatty liver disease, nonalcoholic simple fatty liver, and nonalcoholic steatohepatitis can be identified.

The diagnostic method of the present invention described above can be provided with a software which allows a computer having input means and computing means to function as an apparatus for diagnosing fatty liver disease. Here, input means is an apparatus for inputting the above-described choline concentrations as numerical data which includes, for example, a mouse, a keyboard, and various interfaces. Furthermore, computing means (for example, CPU) in the computer executes the above-described diagnosis of nonalcoholic fatty liver disease and determination of severity thereof by comparing choline concentrations inputted from the input means with reference values. At this time, the computing means reads the reference values from a storage unit in which the reference value are stored, and outputs diagnosis results after comparison with the choline concentration values. A RAM, a hard disk, or the like inside the computer may be used as the storage unit, and it is sufficient that the computer can access the storage unit via a communication network such as Internet or LAN.

Furthermore, the storage unit may be a relational database in which reference values are related to information such as race, sex, age, and past illnesses. In this case, when subject's information such as race, sex, age, and past illnesses is inputted from the input means, the computing means reads a reference value corresponding to the information from the storage means and compares the reference value and the choline concentration.

The computer may output diagnosis results to output means such as a display or a printer. For example, diagnosis results can be outputted to other data processing terminals in the email format.

### Examples

The present invention will be explained more specifically with reference to the following Examples. However, the present invention is not limited thereto.

### Example 1

### Materials and method

### Serum samples

Written informed consent was obtained from patients with NAFLD, NASH, diabetes, chronic hepatitis C, or cirrhosis definitely diagnosed by liver biopsy and various other tests, and blood was collected early in the morning after fasting, and serum was separated.

### Measuring method

Choline was quantified according to the method of Koc, H., et al., (Anal. Che. 2002, 74, 4734-4740) using an HPLC-mass spectrometer. Choline was measured by the isotope dilution method.

### Apparatus models used

Walters Z-MS2000 Electrospray Ionization Mass spectrometer and HP1100 Series HPLC apparatus were used. A mass spectrometer manufactured by Waters Corporation was used, and measurement was performed under the following conditions: total nitrogen gas flow rate, 150 L/min; cone gas flow rate, 50 L/min; ionization voltage, 5 kV; cone voltage, 30 kV; and capillary temperature, 350°C. HPLC was performed using an apparatus manufactured by Waters Corporation, and a usual measuring method was used.

### Standard substance

(N,N,N-trimethyl-d₉)-choline bromide was purchased from CNS Isotope Inc. (Quebec, Canada).

### Pretreatment method

100 µL of a serum sample was placed in a 1.5-mL centrifuge tube, 1 µg of an internal standard sample (deuterated choline) was added, and then 400 µL of methanol/chloroform (2:1, v/v) was added. The precipitates were precipitated at 1500 g for 5 minutes, the supernatant was transferred to a fresh tube. The obtained precipitates were further reextracted by adding 250 µL of methanol/chloroform/water (2:1:0.8, v/v). These two supernatants were combined, 100 µL of chloroform and 100 µL of water were added to the solution, and the mixture was centrifuged to divide into two layers. The aqueous layer portion was used for analysis of choline.

### Analysis of choline

The aqueous layer obtained in the pretreatment procedure was evaporated, the 20 µL of water was further added to dissolve the obtained residue, and then 800 µL of methanol was added. 10 µL of the mixture was used for HPLC analysis. HPLC was performed using Waters Atlantis^{™} HILIC Silica 4.6 x 150 mm (3 µm) as a column. The eluent was composed of solution A: acetonitrile/water/ethanol/1 M ammonium acetate/acetic acid (800:127:68:3:2, v/v) and solution B: acetonitrile/water/ethanol/1 M ammonium acetate/acetic acid (500:500:85:27:18, v/v), and gradient elution was performed with 0% B for 3 minutes, 40% B for 10 minutes, 45% B for 14 minutes, 60% B for 18 minutes, 100% B for 26 minutes. Furthermore, the flow rate was set to be 1.0 mL/min. The column temperature was set to be 40°C. Using the same method, the ion mass of choline was monitored at m/z 104, 113, 118, and 127.

The measurement error of this method was within 8.5% for choline.

### Results

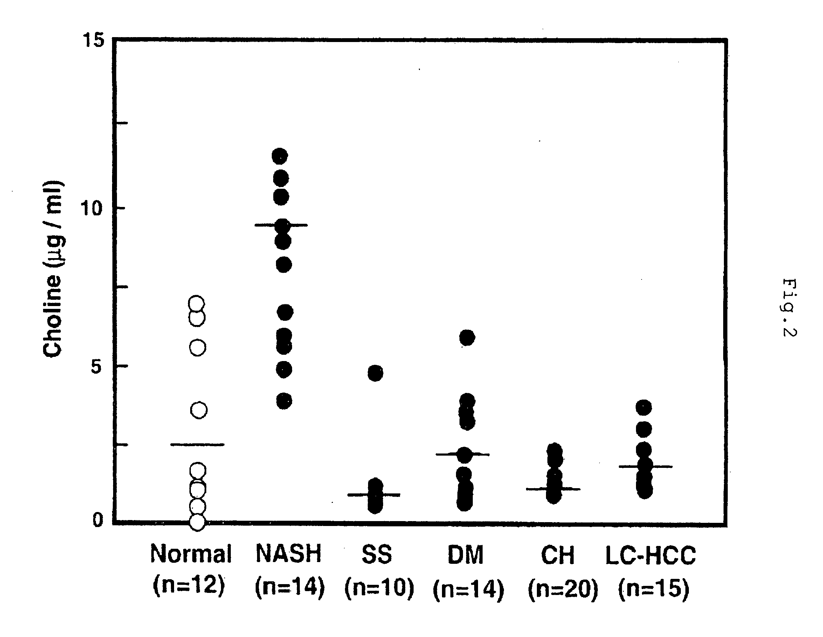

The results are shown in Figure 2. In Figure 2, Normal represents a healthy subject, SS represents patients with NAFLD, DM represents patients with diabetes mellitus, CH represents patients with chronic hepatitis C, LC represents patients with cirrhosis, and LC-HCC represents patients with liver cancer. As shown in this scatter diagram, patients with NASH show significantly higher values as compared with healthy subjects and patients with chronic hepatitis C, cirrhosis, liver cancer, diabetes, or simple fatty liver (NAFLD).

### Discussion

Here, suppose that the choline concentration is measured according to the method of Koc, H., et al., (Anal. Che. 2002, 74, 4734-4740) using an HPLC-mass spectrometer. For example, when the serum choline concentration exceeds 9.5 µg/mL, the presence of steatohepatitis, high severity of the disease, or an inadequate therapeutic effect on the disease can be determined. The positive rate with this cutoff value (9.5 µg/mL) was 2% in healthy subjects, but was high in patients with NAFLD and NASH, with 18% and 68%, respectively. The cutoff value can be obtained by selecting a value so that 98% of normal subjects or patients with diabetes and 50% of patients with NASH show values equal to or lower than this cutoff value.

From the above results, the present inventors found that the serum choline level is useful for diagnosis of NASH, and analyzed the reasons why the serum choline level was high in patients with NASH. Normally, fatty liver and steatohepatitis can be caused by giving choline-deficient feeds in an animal experiment. In humans, however, serum choline levels were high on the contrary. After the following analysis of the reasons, the present inventors concluded that "choline resistance" described below plays an important role in development of clinical conditions of fatty liver and steatohepatitis. The concept of this clinical condition is considered to be an important finding in future consideration of diagnosis and treatment of this disease.

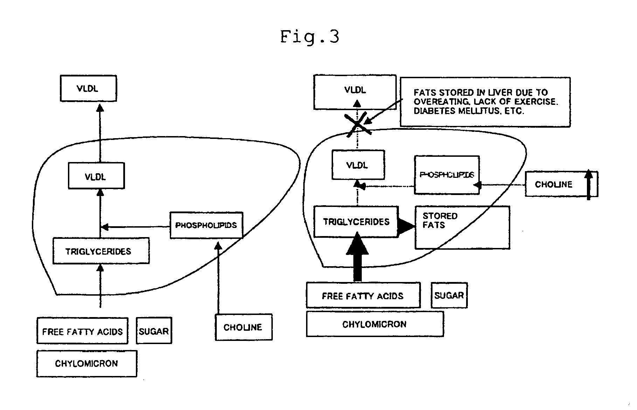

### (Concept of choline resistance [Figure 3])

Choline derived from meal is absorbed from the intestinal tract and converted to phospholipids in the liver. Meanwhile, fats and carbohydrates in a meal are converted to triglycerides in the liver, assembled in the phospholipid membrane as very low density lipoproteins (VLDL), and secreted into blood as carriers of fat-derived energy to peripheral organs. The pathological fat storage in the liver is attributed to lack of exercise and excess energy intake as well as an increased energy inflow into the liver caused by underlying diabetes or the like and a decreased release of fat-derived energy from the liver. As a result, unreleased triglycerides in the liver are stored as lipid droplets. Use of phospholipids is decreased with the decreased release of VLDL, and choline, which serves as a raw material, is not used, resulting in the high blood concentration thereof. Originally, the liver plays an role as an organ for storing and supplying energy between meals. In the case of temporary storage, VLDL are synthesized from triglycerides and phospholipids using choline as a raw material and secreted. Therefore, choline in blood is consumed. That is, it is considered that, even if a large amount of energy is ingested, blood choline levels do not increase while VLDL can be synthesized using choline and secreted. On the other hand, when, although it is also the condition of fatty liver, stored fats are full in the liver, and the stored fats are not secreted or degraded and stay in the liver for a long period, choline is not used, resulting in the increased blood choline concentrations. It is understood that the high serum choline levels found in patients with fatty liver or steatohepatitis by the present inventors are caused by this mechanism. This finding suggests that, so long as stored fats are momentarily turned over even in a condition that a very large amount of fats are stored in the liver, a large amount of choline in blood is consumed, and blood concentrations do not increase. However, if stored fats are hardly turned over in the liver, blood choline levels increase. Fatty liver, a condition in which fats are accumulated in the liver, is classified into a fatty liver type in which the fats are dynamically flows into and released from the liver, resulting in low serum choline levels, and a fatty liver type in which stored fats in the liver do not move, resulting in high choline levels. The analysis results have shown that high choline levels are more often observed in steatohepatitis rather than in simple fatty liver, and pathological conditions of NASH include a condition in which choline cannot be used in the liver in spite of the high choline levels. The present inventors decided to call this condition "choline resistance" as an unprecedented new concept.

### Example 2

### Serum samples

Written informed consent was obtained from healthy subjects, patients with simple fatty liver, and patients with NASH definitely diagnosed by liver biopsy and various other tests, blood was collected early in the morning after fasting, and serum was separated. Furthermore, severity of NASH was determined by the degree of progression of fibrosis based on the results of liver biopsy to classify NASH as F1, F2, F3, or F4. These F1, F2, F3, and F4 correspond to the above-described four stages of Brunt et al. (stages 1 to 4).

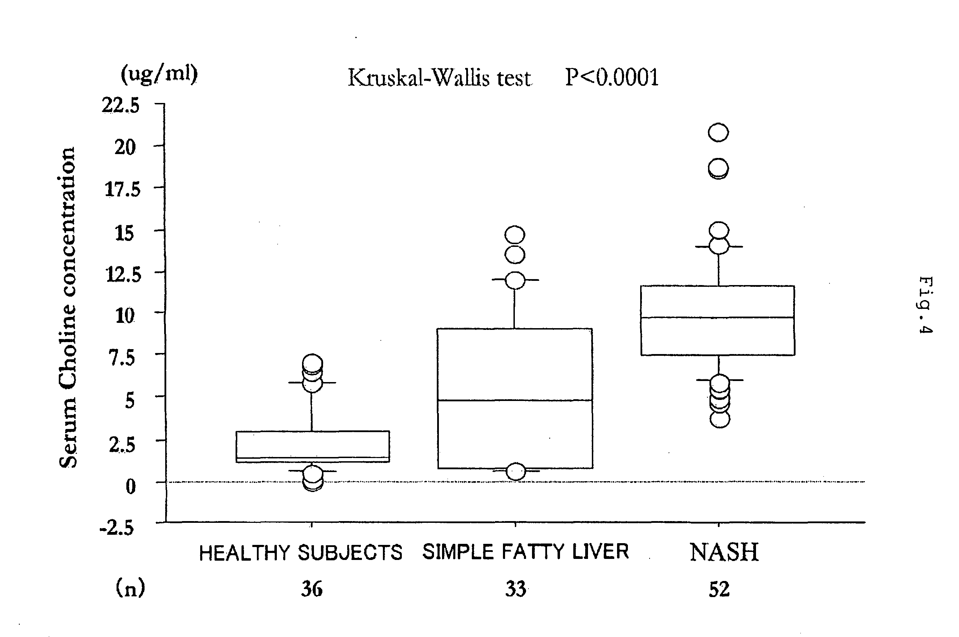

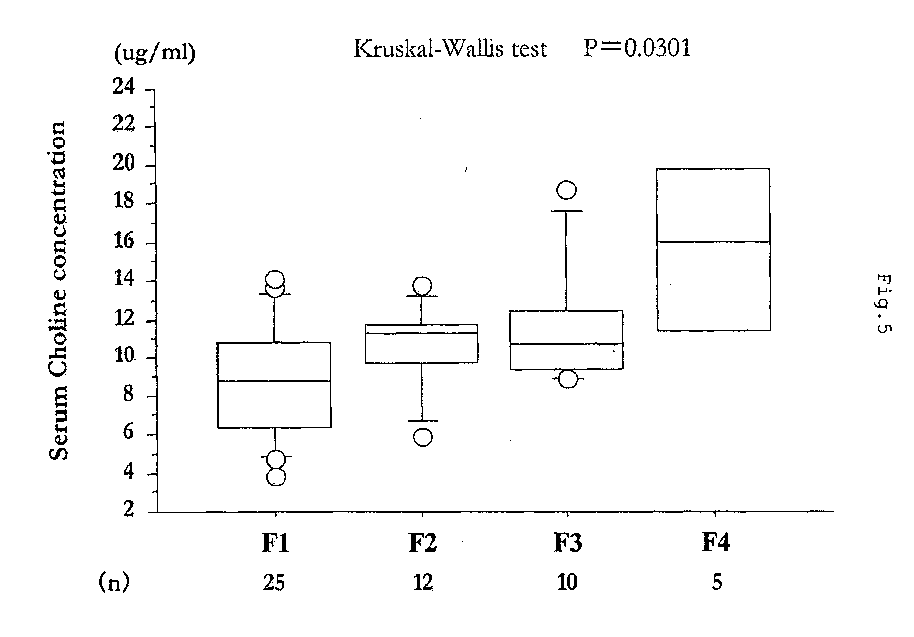

In this Example, the measuring method, the model of the apparatuses used, the standard substance, the pretreatment method, and the choline analysis were the same as in Example 1. The results in this Example are shown in Figures 4 and 5.

As shown in Figure 4, it was found that serum choline concentrations significantly increased in healthy subjects, patients with simple fatty liver, and patients with NASH in this order. These results demonstrated that simple fatty liver and NASH can be diagnosed using the concentration of choline contained in a blood-derived sample as an indicator. Furthermore, as shown in Figure 5, it was found that serum choline concentrations significantly increased depending on severity of NASH, that is, the degree of fibrosis. These results demonstrated that the degree of progression of NASH can be determined using the concentration of choline contained in a blood-derived sample as an indicator.

### Example 3

### Serum samples

Written informed consent was obtained from healthy subjects, patients with simple fatty liver, patients with NASH, and patients with alcoholic hepatitis (ASH) definitely diagnosed by liver biopsy and various other tests, blood was collected early in the morning after fasting, and serum was separated. Furthermore, severity of NASH was determined by the degree of progression of fibrosis based on the results of liver biopsy to classify NASH as F1, F2, or F3. These F1, F2, and F3 correspond to stages 1 to 3, respectively, in the above-described four stages of Brunt et al.

### Measuring method

In this Example, serum choline concentrations were measured using enzyme reagents. Specifically, the serum choline concentration was obtained by allowing a choline oxidase to act on free choline in serum to oxidize it to betaine, oxidatively condensing N-ethyl-N-(3-sulfopropyl)-m-anisidine (ESPAS) and 4-aminoantipyrine (4-AA) with hydrogen peroxide produced at this time by peroxidase to produce a high-sensitivity dye, and measuring the absorbance thereof.

As the enzyme reagents used in this Example, a first reagent and a second reagent having the following compositions were prepared.

### Composition of first reagent

TES (Dojindo Laboratories)-NaOH (Nacalai Tesque Inc.) 50 mM pH 7.1
Ascorbic acid oxidase (Toyobo Co., Ltd. ASO-311) 2 U/mL
Peroxidase (Toyobo Co., Ltd. PEO-301) 4 U/mL
4-Aminoantipyrine (Daiich Pure Chemicals Co., Ltd.) 1.5 mM
TES is N-tris(hydroxymethyl)methyl-2-aminoethanesulfonic acid.

### Composition of second reagent

PIPES (Dojindo Laboratories)-NaOH (Nacalai Tesque Inc.) 50 mM pH 7.5
Choline oxidase (Toyobo Co., Ltd. CHO-301) 20 U/mL N-ethyl-N-(3-sulfopropyl)-m-anisidine (Dojindo Laboratories) 3 mM
PIPES is piperazine-1,4-bis(2-ethanesulfonic acid).

Choline concentrations were measured by the following procedures using the first reagent and the second reagent. First, 150 µL of the first reagent was added to 50 µL of a serum sample, and the mixture was incubated at 37°C for 5 minutes as a first reaction. Subsequently, 50 µL of the second reagent was added, and the mixture was incubated for 15 minutes as a second reaction. Absorbance was measured after completion of the first reaction and completion of the second reaction. The difference between the absorbance after competition of the second reaction and the absorbance after completion of the first reaction was obtained, and then the choline concentration was determined based on the separately prepared calibration curve. Absorbance was measured at a wavelength of 630 nm using a microplate reader Multiscan JX (Thermo Electron Co., Ltd.). To create a calibration curve, 54 mg/dL of choline chloride (standard solution for measuring phospholipids, Toyobo Co., Ltd.) was used as the standard substance of choline.

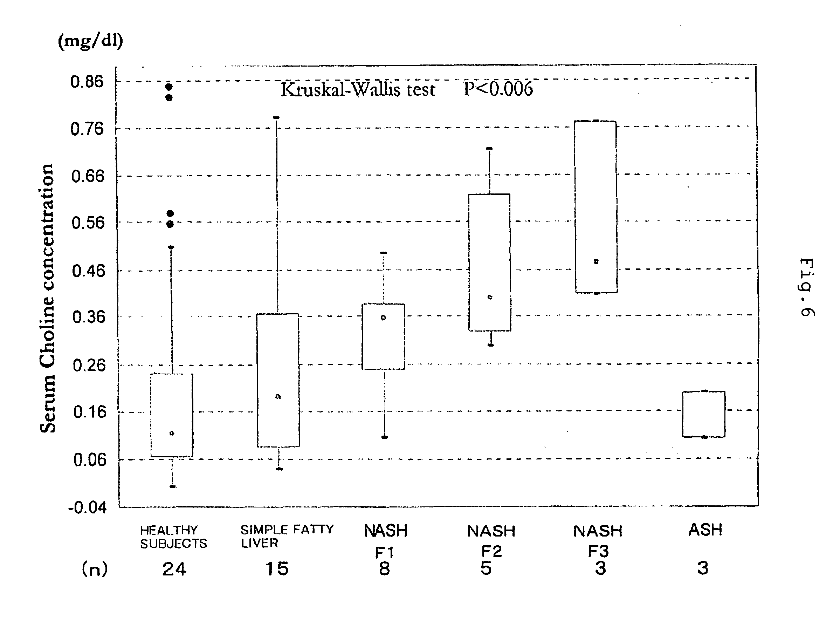

The results of this Example are shown in Figure 6. As shown in Figure 6, it was found that the serum choline concentration increases as symptoms of steatohepatitis become severer from healthy subjects, to patients with simple fatty liver, to patients with NASH at F1, to patients with NASH at F2, and to patients with NASH at F3. Furthermore, it was found that serum choline concentrations in patients with alcoholic clinical condition (ASH) among steatohepatitis are at the same level as those in healthy subjects unlike patients with NASH. The above results demonstrated that, when concentrations of choline contained in blood-derived samples are measured using enzyme reagents, simple fatty liver and NASH can also be diagnosed using the measured value of the choline concentration as an indicator. Furthermore, it was demonstrated that, when concentrations of choline contained in blood-derived samples are measured using enzyme reagents, the degree of progression of NASH can also be diagnosed. Furthermore, the above-described results demonstrated that, when the concentration of choline contained in a blood-derived sample of a patient definitely diagnosed as steatohepatitis by, for example, liver biopsy and various other tests increases, NASH can be diagnosed, and when the choline concentration does not increase, ASH can be diagnosed. That is, it has become clear that NASH or ASH can be diagnosed not by ambiguous criteria such as history taking, but based on objective values such as concentrations of choline contained in blood-derived samples.

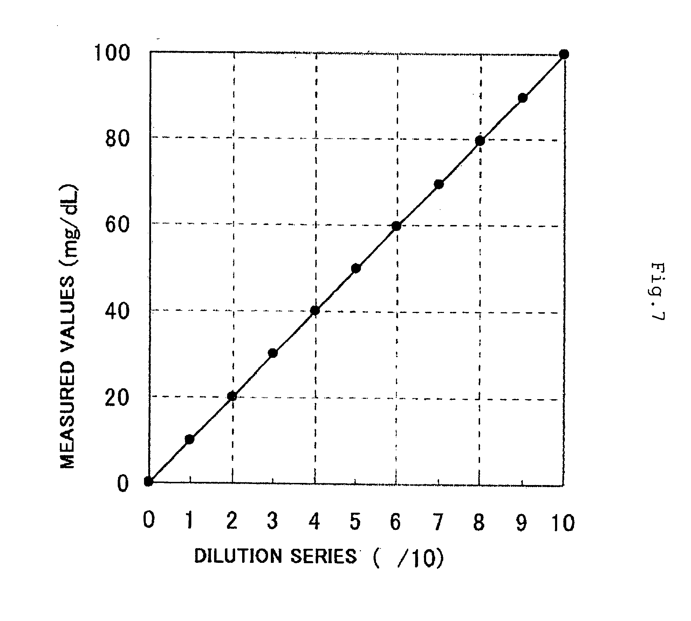

The choline concentration measuring ability of the enzyme reagents used in this Example was evaluated as follows. First, 120 µL of the first reagent was added to 5 µL of a sample, and the mixture was incubated at 37°C for 5 minutes as a first reaction. Then, 60 µL of the second reagent was added, and the mixture was incubated for 5 minutes as a second reaction. Absorbance was measured after completion of the first reaction and completion of the second reaction. Then, absorbance was measured at a dominant wavelength of 600 nm (complementary wavelength, 800 nm) as the measured wavelength in the two point end method in which the absorbance of the first reaction and the absorbance of the second reaction are subjected to fluid volume correction to obtain the difference between them. To measure absorbance, Model 7180 Automatic Analyzer manufactured by Hitachi, Ltd. was used. Here, high concentration choline chloride was added to serum, and dilution series prepared with physiological saline were used as samples. For calibration, 53 mg/dL of aqueous choline chloride was used. When, after measurement, the sample concentrations were calculated and plotted, it was confirmed that measurements up to 100 mg/dL showed linearity (Figure 7).

### Example 4

### Serum samples

Written informed consent was obtained from healthy subjects, patients with simple fatty liver, and patients with NASH definitely diagnosed by liver biopsy and various other tests, blood was collected early in the morning after fasting, and serum was separated.

### Measuring method

In this example, concentrations of choline contained in serum samples were measured using the analyzing apparatus shown in Figure 1. As the HPLC apparatus in the analyzing apparatus, Prominence series HPLC apparatus (Shimadzu Corporation) was used. As the analysis column, TSK Gel Lipopropak XL (Tosoh Corporation) was used. As the reagent for detection, a reagent for measuring phospholipids Liquid PL (Toyobo Co., Ltd.) was used. As the standard substance, 54 mg/dL of choline chloride (standard solution for measuring phospholipids, Toyobo Co., Ltd.) was used. 10 µL of serum was injected, TSK eluent LP-1 or LP-2 was sent as an eluent at a flow rate 0.7 mL/min. Based on the obtained chromatogram, free choline concentrations were obtained by the analyzing program described in Japanese Patent No. 3899041 as the serum choline values. Furthermore, in this Example, the concentrations of choline contained in the same serum samples were measured using enzyme reagents in the same manner as in Example 3.

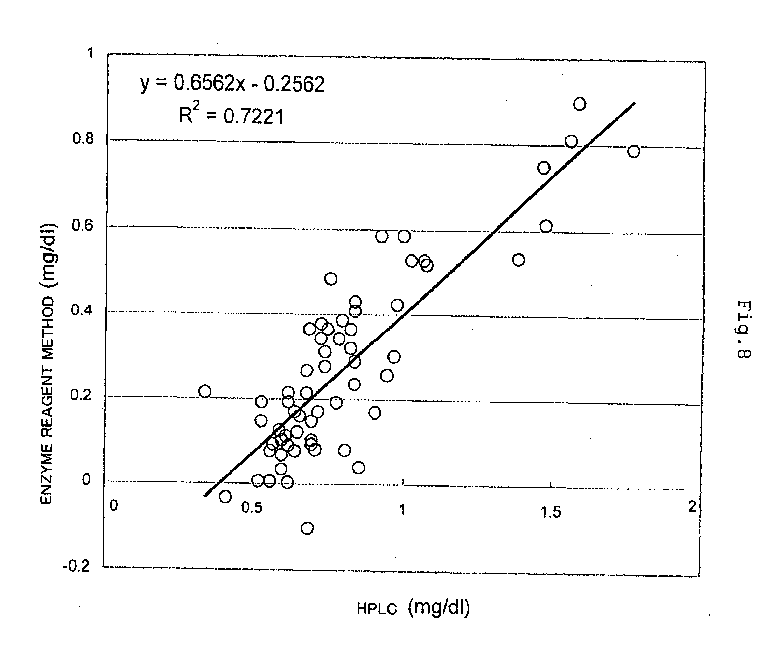

Figure 8 shows the results of measurement of choline concentrations using the analyzing apparatus shown in Figure 1 and the results of measurement of choline concentrations using the enzyme reagents of Example 3. As shown in Figure 8, it was demonstrated that the values of choline concentrations measured using enzyme reagents and the values of choline concentrations measured by the HPLC apparatus closely correlated (R² = 0.7221). This result demonstrated that concentrations of choline contained in blood-derived samples can also be measured using the analyzing apparatus shown in Figure 1 with precision comparable to that of the measurement using enzyme reagents of Example 3. Therefore, it was demonstrated that, by measuring concentrations of choline contained in blood-derived samples using the analyzing apparatus shown in Figure 1, diagnosis of simple fatty liver and NASH, determination of the degree of progression of NASH, and further diagnosis of NASH or ASH in a patient definitely diagnosed as steatohepatitis by liver biopsy and various other tests can be achieved correctly using the measured choline concentration as an indicator.

### Industrial Applicability

The present invention demonstrated that choline is useful as a serum diagnostic marker of NAFLD and steatohepatitis. Therefore, choline can be used as a serum diagnostic marker to diagnose NAFLD and steatohepatitis and determine a therapeutic effect on these diseases.

## Claims

1. A method for diagnosing non alcoholic fatty liver disease, comprising the steps of (a) measuring the concentration of choline contained in a blood-derived sample collected from a subject; and (b) determining the presence of non alcoholic fatty liver disease, based on the choline concentration, wherein a subject is classified as a patient with non alcoholic fatty liver disease based on the presence of an increased choline concentration during the determination step when compared to the choline concentration in a blood-derived sample from a healthy subject or from a patient with alcoholic steatohepatitis.

2. The method according to claim 1, **characterized in that** the blood-derived sample is a serum sample, and the choline concentration is the concentration of free choline in serum.

3. The method according to claim 1, **characterized in that** the choline concentration is measured by high performance liquid chromatography, liquid chromatography-mass spectrometry, gas chromatography-mass spectrometry, use of enzyme reagents including choline esterase, use of a reagent which chemically reacts with choline, electrophoresis, nuclear magnetic resonance, ultracentrifugation, spectroscopy using an ultraviolet ray, or measurement of a potential difference.

4. The method according to any one of claims 1 to 3, wherein a subject is classified as a patient with non alcoholic fatty liver disease based on the presence of a choline concentration significantly exceeding the mean + confidence interval of the choline concentration in a blood-derived sample from a normal individual.

5. The method according to claim 1, **characterized in that** the degree of progression of non alcoholic fatty liver disease in the subject is determined based on the choline concentration during the determination step.

6. The method according to any one of the preceding claims wherein said non alcoholic fatty liver disease is nonalcoholic steatohepatitis. -

7. The method according to claim 1, **characterized in that** the subject is classified as a patient with non alcoholic steatohepatitis and is further classified for the degree of progression of non alcoholic steatohepatitis based on the choline concentration.

8. The method according to claim 1, **characterized in that** the subject is classified as a patient with non alcoholic fatty liver disease, non alcoholic simple fatty liver, or non alcoholic steatohepatitis based on the choline concentration during the determination step.

9. The method according to claim 1, **characterized in that** the subject is suspected of having steatohepatitis and is classified as a patient with non alcoholic steatohepatitis based on the presence of an increased choline concentration during the determination step when compared to the choline concentration in a blood-derived sample from a healthy subject or from a patient with alcoholic steatohepatitis.

10. A method for screening for a therapeutic agent for non alcoholic fatty liver disease, comprising the steps of:
comparing the concentration of choline contained in a blood sample from a subject before and after a test substance has been administered; and
identifying a test substance which significantly decreases the choline concentration as a therapeutic agent for non alcoholic fatty liver disease.

## Patentansprüche

1. Verfahren zur Diagnose nicht-alkoholbedingter Fettlebererkrankung, umfassend die Stufen (a) Messen der Konzentration an Cholin, das enthalten ist in einer vom Blut abgeleiteten Probe, die von einer Person genommen wurde; und (b) das Feststellen des Vorliegens von nicht-alkoholbedingter Fettlebererkrankung auf der Grundlage der Cholinkonzentration, wobei eine Person als Patient mit nicht-alkoholbedingter Fettlebererkrankung klassifiziert wird auf der Grundlage des Vorhandenseins einer erhöhten Cholinkonzentration während der Stufe des Feststellens, wenn verglichen wird mit der Cholinkonzentration in einer vom Blut abgeleiteten Probe einer gesunden Person oder von einem Patienten mit alkoholbedingter Steatohepatitis.

2. Verfahren nach Anspruch 1, **dadurch gekennzeichnet, dass** die vom Blut abgeleitete Probe eine Serumprobe ist und die Cholinkonzentration die Konzentration an freiem Cholin in dem Serum ist.

3. Verfahren nach Anspruch 1, **dadurch gekennzeichnet, dass** die Cholinkonzentration gemessen wird durch Hochleistungsflüssigkeitschromatographie, Flüssigkeitschromatographie-Massenspektrometrie, Gaschromatographie-Massenspektrometrie, Verwendung von Enzymreagenzien, einschließlich Cholinesterase, Verwendung eines Reagenses, das chemisch mit Cholin reagiert, Elektrophorese, kernmagnetische Resonanz, Ultrazentrifugation, Spektroskopie unter Verwendung ultravioletter Strahlung oder Messung einer Potentialdifferenz.

4. Verfahren nach einem der Ansprüche 1 bis 3, wobei eine Person als Patient mit nicht-alkoholbedingter Fettlebererkrankung klassifiziert wird auf der Grundlage des Vorhandenseins einer Cholinkonzentration, die den Mittelwert + Konfidenzintervall der Cholinkonzentration in einer vom Blut abgeleiteten Probe einer normalen Person signifikant übersteigt.

5. Verfahren nach Anspruch 1, **dadurch gekennzeichnet, dass** der Grad des Fortschreitens der nicht-alkoholbedingten Fettlebererkrankung bei der Person bestimmt wird auf der Grundlage der Cholinkonzentration während der Stufe des Feststellens.

6. Verfahren nach einem der voranstehenden Ansprüche, wobei die nicht-alkoholbedingte Fettlebererkrankung nicht-alkoholbedingte Steatohepatitis ist.

7. Verfahren nach Anspruch 1, **dadurch gekennzeichnet, dass** die Person als Patient mit nicht-alkoholbedingter Steatohepatitis klassifiziert wird und des weiteren bezüglich des Grads des Fortschreitens der nicht-alkoholbedingten Steatohepatitis klassifiziert wird auf der Grundlage der Cholinkonzentration.

8. Verfahren nach Anspruch 1, **dadurch gekennzeichnet, dass** die Person klassifiziert wird als Patient mit nicht-alkoholbedingter Fettlebererkrankung, nicht-alkoholbedingter einfacher Fettleber oder nicht-alkoholbedingter Steatohepatitis auf der Grundlage der Cholinkonzentration während der Stufe des Feststellens.

9. Verfahren nach Anspruch 1, **dadurch gekennzeichnet, dass** bei der Person der Verdacht besteht, dass sie Steatohepatitis hat, und diese klassifiziert wird als Patient mit nicht-alkoholbedingter Steatohepatitis auf der Grundlage des Vorhandenseins einer erhöhten Cholinkonzentration während der Stufe des Feststellens, wenn verglichen wird mit der Cholinkonzentration in einer vom Blut abgeleiteten Probe von einer gesunden Person oder von einem Patienten mit alkoholbedingter Steatohepatitis.

10. Verfahren zum Screenen auf ein therapeutisches Mittel für nicht-alkoholbedingte Fettlebererkrankung, umfassend die Stufen:
Vergleichen der Konzentration an Cholin, das enthalten ist in einer Blutprobe von einer Person, bevor und nachdem eine Testsubstanz verabreicht worden ist; und
Identifizieren einer Testsubstanz mit signifikanter Verringerung der Cholinkonzentration als therapeutisches Mittel für nicht-alkoholbedingte Fettlebererkrankung.

## Revendications

1. Procédé de diagnostic d'une maladie du foie gras non alcoolique, comprenant les étapes consistant à (a) mesurer le taux de choline contenue dans un échantillon dérivé du sang prélevé chez un sujet ; et (b) déterminer la présence d'une maladie du foie gras non alcoolique, d'après le taux de choline, dans lequel un sujet est classé en tant que patient ayant une maladie du foie gras non alcoolique en fonction de la présence d'un taux de choline élevé au cours de l'étape de détermination lorsque l'on le compare avec le taux de choline dans un échantillon dérivé du sang provenant d'un sujet sain ou d'un patient présentant une stéatose hépatique alcoolique.

2. Procédé selon la revendication 1, **caractérisé en ce que** l'échantillon dérivé du sang est un échantillon de sérum, et le taux de choline est le taux de choline libre dans le sérum.

3. Procédé selon la revendication 1, **caractérisé en ce que** le taux de choline est mesuré par chromatographie liquide haute performance, spectrométrie de masse couplée à la chromatographie liquide, spectrométrie de masse couplée à la chromatographie gazeuse, par l'utilisation de réactifs enzymatiques comprenant la cholinestérase, par l'utilisation d'un réactif qui réagit chimiquement avec la choline, par électrophorèse, par résonance magnétique nucléaire, par ultracentrifugation, par spectroscopie à rayonnement ultraviolet, ou par mesure d'une différence de potentiel.

4. Procédé selon l'une quelconque des revendications 1 à 3, dans lequel un sujet est classé en tant que patient présentant une maladie du foie gras non alcoolique en fonction de la présence d'un taux de choline excédant de manière significative la moyenne + l'intervalle de confiance du taux de choline dans un échantillon dérivé du sang provenant d'un individu normal.

5. Procédé selon la revendication 1, dans lequel le degré de progression d'une maladie du foie gras non alcoolique chez le sujet est déterminé en fonction du taux de choline au cours de l'étape de détermination.

6. Procédé selon l'une quelconque des précédentes revendications, dans lequel ladite maladie du foie gras non alcoolique est une stéatose hépatique non alcoolique.

7. Procédé selon la revendication 1, **caractérisé en ce que** le sujet est classé en tant que patient présentant une stéatose hépatique non alcoolique et est en outre classé selon le degré de progression de la stéatose hépatique non alcoolique en fonction du taux de choline.

8. Procédé selon la revendication 1, **caractérisé en ce que** le sujet est classé en tant que patient ayant une maladie du foie gras non alcoolique, un foie gras simple non alcoolique, ou une stéatose hépatique non alcoolique en fonction du taux de choline au cours de l'étape de détermination.

9. Procédé selon la revendication 1, **caractérisé en ce que** le sujet est suspecté de présenter une stéatose hépatique et est classé en tant que patient présentant une stéatose hépatique non alcoolique en fonction de la présence d'un taux élevé de choline au cours de l'étape de détermination lorsque l'on le compare avec le taux de choline dans un échantillon dérivé du sang provenant d'un sujet sain ou d'un patient ayant une stéatose hépatique alcoolique.

10. Procédé de criblage d'un agent thérapeutique pour une maladie du foie gras non alcoolique, comprenant les étapes consistant à :
comparer le taux de choline contenue dans un échantillon de sang d'un sujet avant et après qu'une substance d'essai a été administrée ; et
identifier une substance d'essai qui diminue de manière significative le taux de choline en tant qu'agent thérapeutique pour une maladie du foie gras non alcoolique.
